# FASCICULE DE BREVET EUROPEEN

(11) **EP 0 771 557 B1**
(45) Date de publication et mention de la délivrance du brevet: **18.08.1999**
(21) Numéro de dépôt: 96401907.9
(22) Date de dépôt: 05.09.1996
(51) Int. Cl.: A61K 7/48, A61K 7/06

(54) **Utilisation de l'acide ascorbique comme actif pour le traitement de la séborrhée dans une composition cosmétique et/ou dermatologique**
Verwendung von Ascorbinsäure als Wirkstoff zur Behandlung von Seborrhoe in einer kosmetischen und/oder dermatologischen Zusammensetzung
Use of ascorbic acid as the active agent for the treatment of seborrhea in a cosmetic and/or dermatalogical composition

(30) Priorité: 19.09.1995 FR 9510978
(43) Date de publication de la demande: 07.05.1997
(73) Titulaire: L'OREAL, 75008 Paris (FR)
(72) Inventeur: Fanchon, Chantal, 75015 Paris (FR); Gagnebien, Didier, 92320 Chatillon (FR); Cantin, Hervé, 91420 Morangis (FR)
(74) Mandataire: Lhoste, Catherine

(56) Documents cités:
- EP-A- 0 202 621
- EP-A- 0 280 606
- EP-A- 0 447 318
- EP-A- 0 599 819
- EP-A- 0 667 145
- CN-A- 1 080 181
- DE-A- 3 514 724
- DE-A- 4 305 460
- DE-A- 4 328 871
- DE-A- 4 419 783
- FR-A- 2 477 871
- CHEMICAL ABSTRACTS, vol. 121, no. 4, 25 Juillet 1994 Columbus, Ohio, US; abstract no. 42747, XP002005997 & CN 1 080 181 A (STAFF AND WORKERS HOSPITAL OF HENAN PETROLEUM PROSPECTING BUREAU) 5 Janvier 1994
- CHEMICAL ABSTRACTS, vol. 113, no. 8, 20 Août 1990 Columbus, Ohio, US; abstract no. 65292, XP002005998 & JP 02 049 714 A (SHISEIDO CO.) 20 Février 1990
- Epstein, E. (ed.) (1994). Common skin disorders. WB Saunders, pp. 94-97
- Murphy, G.F. (1995). Dermatopathology. WB Saunders, pp. 57-58

## Description

La présente invention a pour objet l'utilisation de l'acide ascorbique comme actif pour traiter la séborrhée

La séborrhée ou hypersécrétion sébacée est à l'origine des troubles cutanés que sont la peau grasse et le cuir chevelu gras, désordres esthétiques qui peuvent devenir le lit d'affections dermatologiques comme l'acné et la dermite séborrhéique.

L'acné est l'une des maladies affectant le plus souvent, et à des degrés divers, la population juvénile entre 15 et 30 ans. L'acné est essentiellement la conséquence de deux phénomènes intriqués : l'hypersécrétion sébacée et la perturbation de la kératinisation du follicule pilosébacé, dont les conséquences sont l'obstruction du follicule pilosébacé et la formation de lésions rétentionnelles ou comédons. Les comédons, par suite de la prolifération microbienne, peuvent évoluer en lésions inflammatoires, papules et pustules. L'acné se manifeste fréquemment au moment de la puberté, mais peut se poursuivre aussi à l'âge adulte et notamment chez les femmes pour des causes hormonales.

L'acné dû à l'hypersécrétion sébacée et à la perturbation de la kératinisation du follicule pilosébacé est une affection différente de ce que l'on appelle l'acné de Majorque (en Anglais 〈〈Mallorca acne〉〉) ou *acne aestivalis*, qui est une photodermatose induite par le soleil et causée par une hypersensibilité de la peau à la lumière et non à une hypersécrétion sébacée. Il d'ailleurs connu par la demande de brevet allemand DE-A-4328871 que cette photodermatose (〈〈Mallorca acne〉〉 ou *acne aestivalis*) peut être traitée par l'utilisation de composés antioxydants comme par exemple l'acide ascorbique.

La dermite séborrhéique est associée à la prolifération de levures du genre *Pityrosporum* sur le substrat que constitue pour elles le sébum.

Dans le cas de l'acné comme dans celui de la dermite séborrhéique, la régulation du flux sébacé supprime la cause initiale, c'est-à-dire la présence excessive de sébum, et traite par voie de conséquence la dermatose.

C'est ce que l'on observe avec l'isotrétinoïne dont l'administration par voie orale induit un assèchement des follicules sébacés et conduit à la disparition des symptômes.

Mais l'utilisation de l'isotrétinoïne n'est pas dénuée d'effets secondaires sérieux et reste de ce fait réservée au traitement des acnés sévères, invalidantes. Les traitements de la séborrhée par voie topique se sont avérés jusqu'à présent peu efficaces et on pallie fréquemment ce manque d'efficacité par des traitements par voie systémique, notamment avec l'isotrétinoïne ou des anti-androgènes.

On constate donc que subsiste le besoin d'actifs topiques ayant un effet sur l'hypersécrétion sébacée et par voie de conséquence sur les dermatoses qui lui sont associées.

C'est ce qu'apporte la présente invention.

La demanderesse a découvert que l'acide ascorbique (ou vitamine C) présentait des propriétés anti-séborrhéiques et pouvait donc être utilisé dans une composition cosmétique ou dermatologique en tant qu'actif pour lutter contre la séborrhée de la peau ou du cuir chevelu, et contre les dermatoses qui peuvent en découler comme l'acné et la dermite séborrhéique.

Les propriétés antioxydantes de l'acide ascorbique sont largement décrites dans l'art antérieur. A ce titre on peut citer les demandes de brevet EP-A-0 667 145, DE-A-43 28 871, CN-A-1 080 181, FR-A-2 477 871, DE-A-3 514 724, EP-A-0 202 621, EP-A-0 280 606

On sait que l'acide ascorbique est déjà utilisé dans les compositions topiques car il a un effet bénéfique sur la peau. En particulier, il stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau (JP-A-02049714) et possède une fonction anti-radicaux libres. L'acide ascorbique est décrit comme composé complexant au même titre que l'éthylène diamine tetraacétate (EDTA) (DE-A-4 305 460). L'acide ascorbique est enfin décrit comme promoteur de l'activité d'actifs dans des compositions cosmétiques ou pharmaceutiques (EP-A-0 599 819).

Toutefois, son utilisation pour traiter la séborrhée n'a jamais été décrite.

La présente invention a donc pour objet l'utilisation de l'acide ascorbique sous forme libre, estérifiée ou éthérifiée, dans et/ou pour la fabrication d'une composition cosmétique et/ou dermatologique, comme actif pour traiter la séborrhée de la peau et/ou du cuir chevelu,

La présente invention a aussi pour objet un procédé de traitement cosmétique et/ou dermatologique de la séborrhée consistant à appliquer sur la peau et/ou le cuir chevelu, une composition contenant l'acide ascorbique comme seul agent de traitement de la séborrhée, et plus particulièrement une composition telle que décrite par la présente invention.

La composition contenant l'acide ascorbique a de préférence un pH allant de 2 à 5, et plus particulièrement un pH d'environ 4. L'acide ascorbique peut être utilisé tel quel ou sous une forme stabilisée, par exemple sous la forme d'un dérivé, ou sous forme de poudre.

Selon une forme de réalisation de l'invention, la composition se présente sous forme d'un support et d'un solide distincts, destinés à être mélangés extemporanément, le solide consistant essentiellement en de l'acide ascorbique pulvérulent. Un tel agencement est décrit par exemple dans la demande FR-94-03982, déposée par la demanderesse.

Les dérivés utilisables sont par exemple ceux obtenus par blocage du site hydroxyle, notamment par estérification ou éthérification avec, par exemple, des composés phosphatés, sulfatés ou alkylés. Il peut s'agir notamment de l'ascorbylphosphate de magnésium, de la vitamine C glycosylée, de l'acétate d'ascorbyle et du palmitate d'ascorbyle.

L'acide ascorbique est utilisé, selon la présente invention, en une quantité allant de préférence de 1 à 20 % en poids et encore plus préférentiellement de 3 à 10 % en poids par rapport au poids total de la composition.

Les compositions selon l'invention peuvent se présenter sous toutes les formes appropriées pour une application topique, notamment sous forme de solutions aqueuses, hydroalcooliques ou huileuses, de dispersions du type lotion ou sérum, de gels aqueux, anhydres ou huileux, d'émulsions de consistance liquide ou semi-liquide du type lait, obtenues par dispersion d'une phase grasse dans une phase aqueuse (H/E) ou inversement (E/H), de suspensions ou d'émulsions de consistance molle, semi-solide ou solide du type crème, gel, de microémulsions, ou encore de microcapsules, de microparticules, ou de dispersions vésiculaires de type ionique et/ou non ionique. Ces compositions sont préparées selon les méthodes usuelles bien connues de l'homme de l'art du domaine.

Les compositions de l'invention peuvent comprendre les adjuvants classiquement mis en oeuvre dans les domaines considérés comme les corps gras, les solvants organiques, les épaississants, les adoucissants, les antioxydants, les opacifiants, les stabilisants, les agents anti-mousse, les parfums, les émulsionnants ioniques ou non, les charges, les séquestrants, les colorants ou tout autre ingrédient habituellement utilisé en cosmétique.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et éventuellement le coémulsionnant sont présents, dans la composition, en une proportion allant de 0,3 à 30 % en poids, et de préférence de 0,5 à 20 % en poids par rapport au poids total de la composition. L'émulsion peut, en outre, contenir des vésicules lipidiques.

Comme huiles utilisables dans l'invention, on peut citer les huiles minérales (huile de vaseline), les huiles végétales (fraction liquide de beurre de karité), les huiles animales, les huiles de synthèse (huile de purcellin, polyisobutène hydrogénée), les huiles siliconées et les huiles fluorées (perfluoropolyéthers). On peut aussi utiliser comme matières grasses des alcools gras, des acides gras, des cires.

Comme émulsionnants utilisables dans l'invention, on peut citer par exemple les esters d'acide gras et de polyol, tels que les esters gras de sorbitol, comme le tristéarate de sorbitan vendu sous la dénomination de Span 65® par la société ICI, ou aussi les esters gras de glycérol tels que le monostéarate de glycérol, ou encore les esters de PEG tels que le stéarate de PEG-40 vendu sous la dénomination de Myrj 52® par la société ICI. Il peut s'agir aussi d'émulsionnants siliconés tels que le cétyl diméthicone copolyol vendu sous la dénomination d'Abil EM90® par la société Goldschmidt.

Comme gélifiants hydrophiles, on peut citer les polymères carboxyvinyliques (carbomer), les copolymères acryliques tels que les copolymères d'acrylates/alkylacrylates, les poly(méth)acrylates de glycéryle tels que le produit vendu sous la dénomination de Norgel® par la société Guardian, les polyacrylamides et notamment le mélange de polyacrylamide, C13-14-Isoparaffine et Laureth-7, vendu sous la dénomination de Sepigel 305® par la société Seppic, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Les compositions de l'invention peuvent comprendre aussi des actifs, hydrophiles ou lipophiles, et notamment les actifs susceptibles de compléter l'effet de l'acide ascorbique dans le traitement de la séborrhée et des dermatoses associées, et notamment de l'acné. Il peut s'agir par exemple d'agents anti-inflammatoires tels que le peroxyde de benzoyle, d'antibiotiques, d'agents antiseptiques tels que l'octopirox ou d'actifs kératolytiques tels que l'acide salicylique et ses dérivés, les alpha-hydroxyacides, l'acide rétinoïque et ses dérivés, le rétinol et ses dérivés.

Par ailleurs, comme actifs hydrophiles, on peut utiliser par exemple les protéines ou les hydrolysats de protéine, les acides aminés, les polyols (glycérine, propylène glycol), l'urée, l'allantoïne, les sucres et les dérivés de sucre, les vitamines hydrosolubles, l'amidon, les extraits bactériens ou végétaux, notamment d'Aloe Vera, les agents hydratants.

Comme actifs lipophiles, on peut utiliser par exemple le tocophérol (vitamine E) et ses dérivés, les acides gras essentiels, les céramides, les huiles essentielles.

Les exemples suivants illustrent l'invention sans en limiter aucunement la portée. Les pourcentages sont donnés en poids.

### Exemple 1 : Crème sous forme d'émulsion huile-dans-eau

| *Phase huileuse :* | |
|---|---|
| - Abil EM90® (émulsionnant) | 3 % |
| - Silicone volatile | 12 % |
| - Huile de purcellin | 3 % |
| - Gel de bentone | 5 % |
| - Fraction liquide de beurre de karité | 2 % |

| *Phase aqueuse :* | |
|---|---|
| - Sel de sodium de l'EDTA (séquestrant) | 0,1 % |
| - Acide ascorbique | 5 % |
| - Eau | qsp 100% |

Le procédé de préparation de l'émulsion consiste à préparer la phase grasse et à l'introduire dans la phase aqueuse sous agitation.

La crème obtenue convient pour le traitement de la dermite séborrhéique, en application une à deux fois par jour sur le visage.

### Exemple 2 : Gel

| | |
|---|---|
| - Polyacrylate de glycéryle (Norgel®) | 29,5 % |
| - Polyacrylamide/C13-14 Isoparaffine/laureth-7 (Sepigel 305®) | 2 % |
| - Huile de silicone | 10 % |
| - Acide ascorbique | 5 % |
| - Sel de sodium de l'EDTA (séquestrant) | 0,1 % |
| - Conservateur | 0,4 % |
| - Eau | qsp 100 % |

Le procédé de préparation du gel consiste à mélanger dans l'eau le polyacrylate de glycéryle, le Sepigel 305®, le séquestrant et l'acide ascorbique, puis à introduire sous agitation l'huile de silicone.

Le gel obtenu convient pour le traitement de la peau grasse, par application sur le visage une ou deux fois par jour.

### Exemple 3 : Crème sous forme d'émulsion H/E

| *Phase huileuse :* | |
|---|---|
| - Span 65® | 0,9 % |
| - Monostéarate de glycéryle | 3 % |
| - Polyisobutène hydrogénée | 6 % |
| - Huile de silicone volatile | 7 % |
| - Stéarate de PEG-41 (Myrj 52®) | 2 % |

| *Phase aqueuse :* | |
|---|---|
| - Polyacrylamide/C13-14 Isoparaffine/laureth-7 (Sepigel 305®) | 0,9 % |
| - Sel de sodium de l'EDTA (séquestrant) | 0,05 % |
| - Conservateur | 0,5 % |
| - Glycérine | 3 % |
| - Acide ascorbique | 8 % |
| - Eau | qsp 100 % |

Le procédé de préparation de l'émulsion consiste à disperser la phase huileuse dans la phase aqueuse sous agitation à l'homogénéisateur.

La crème obtenue convient pour le traitement de l'acné par application une à deux fois par jour sur le visage.

Le test ci-dessous met en évidence l'effet anti-séborrhéique de l'acide ascorbique. Le gel utilisé pour ce test avait la composition suivante (pH=4) :

| | |
|---|---|
| - Acide ascorbique | 5 % |
| - Sel de sodium de l'EDTA (séquestrant) | 0,1 % |
| - Extrait glycolique de feuilles d'hamamélis | 0,47 % |
| - Hydroxyde de sodium | 0,83 % |
| - Acide citrique, 1 H₂O | 1,24 % |
| - Carraghénane (gélifiant) | 1,26 % |
| - Chlorure de guar hydroxypropyl tri-méthylammonium (gélifiant) | 0,47 % |
| - Polymère acrylique dans eau/glycérine à 1 % (gélifiant) | 28,5 % |
| - Isoprène glycol | 4,75 % |
| - Conservateur | 0,48 % |
| - Eau | qsp 100% |

Le test a été réalisé sur 3 groupes comportant chacun 20 volontaires des deux sexes (60 sujets au total), âgés de 18 à 25 ans. Un groupe de 20 volontaires a appliqué le gel contenant l'acide ascorbique sur tout le visage deux fois par jour (matin et soir) pendant deux mois et les deux autres groupes ont appliqué de la même manière des gels placebo, l'un de pH 3, l'autre de pH neutre.

On a évalué la sécrétion sébacée au début de l'expérience (T0), après 6 semaines de traitement (T6) et après 8 semaines de traitement (T8) par 〈〈sébutapes〉〉 et par évaluation subjective. Certains volontaires ayant abandonné le traitement, le test a été réalisé sur 18 personnes pour le gel contenant l'acide ascorbique et 19 personnes pour le gel de pH neutre.

Les 〈〈sébutapes〉〉 sont des patchs collecteurs de sébum, permettant de suivre et de quantifier *in vivo* l'activité des glandes sébacées et leur sécrétion. Ils sont constitués d'un film de polymère poreux, recouvert d'un film adhésif qui permet un contact intime entre la peau et le patch. A l'embouchure de chaque follicule sébacé, les lipides sécrétés vont traverser le film adhésif et remplir les micropores du film polymère. Ils vont ainsi provoquer l'apparition de taches translucides contrastant avec le film blanc. L'activité sébacée est déterminée par évaluation du nombre de taches qui correspond au nombre de glandes sébacées, selon un score de 0 à 4, 0 étant le score quand le nombre de taches est quasiment nul et 4 étant le score quand le nombre de taches est très important. Cette technique permet d'évaluer l'action antiséborrhéique d'un produit.

Le test au 〈〈sébutape〉〉 est réalisé selon le protocole suivant : on nettoie le front à l'alcool à 70°, puis on tamponne et sèche et on applique le 〈〈sébutape〉〉. On laisse 20 minutes et on retire ensuite le 〈〈sébutape〉〉 dont on détermine alors le score.

Le tableau ci-dessous donne le nombre de personnes pour chaque score entre 0 et 4 :

| | Gel à l'acide ascorbique | | | Gel de pH 3 | | | Gel de pH neutre | | |
|---|---|---|---|---|---|---|---|---|---|
| Scores | T0 | T6 | T8 | T0 | T6 | T8 | T0 | T6 | T8 |
| 0 | | | 1 | | | | | | |
| 1 | | 9 | 9 | | 6 | 5 | | 8 | 7 |
| 2 | 11 | 6 | 5 | 10 | 9 | 12 | 14 | 8 | 11 |
| 3 | 6 | 3 | 3 | 10 | 4 | 3 | 5 | 3 | 1 |
| 4 | 1 | | | | | | | | |

L'analyse de la fréquence des scores (test de Friedman) a mis en évidence une diminution significative de la production de sébacée dès 6 semaines par rapport à T0 pour le gel à l'acide ascorbique et pour le gel à pH 3, les résultats étant significativement meilleurs pour le gel à la vitamine C (Score de 0 et 1 pour 10 personnes ayant utilisé le gel à l'acide ascorbique contre seulement 5 personnes ayant utilisé le gel à pH 3).

Par ailleurs, les volontaires du test ont fait une évaluation subjective de leur peau après application des gels, sur une échelle de 9 points allant du 〈〈très gras〉〉 au 〈〈peu gras〉〉. Le tableau ci-dessous rend compte du score moyen obtenu pour les volontaires de chaque groupe et de la différence entre le score à T0 et le score à T6 ou T8 :

| Gel | T0 | T6 | T0 - T6 (%) | T8 | T0 - T8 (%) |
|---|---|---|---|---|---|
| Gel à l'acide ascorbique | 6,3 | 4,6 | - 27 % | 3,9 | - 38 % |
| Gel de pH 3 | 5,7 | 5,1 - | 10,5 % | 4 | - 30 % |
| Gel de pH neutre | 5,95 | 5,42 | - 9 % | 5,05 | - 15 % |

Ces résultats montrent que l'application du gel contenant de l'acide ascorbique entraîne une diminution significative de la peau grasse dès 6 semaines par rapport à T0.

## Revendications

1. Procédé de traitement cosmétique de la séborrhée, consistant à appliquer sur la peau et/ou le cuir chevelu, une composition contenant de l'acide ascorbique comme seul agent de traitement de la séborrhée.

2. Procédé selon la revendication 1, caractérisé en ce que l'acide ascorbique est sous forme libre.

3. Procédé selon l'une quelconque des revendications 1 ou 2, caractérisée en ce que la composition a un pH allant de 2 à 5.

4. Procédé selon l'une quelconque des revendications précédentes, caractérisée en ce que la composition a un pH de 4.

5. Procédé selon l'une quelconque des revendications précédentes, caractérisée en ce que l'acide ascorbique est mis en oeuvre en une quantité allant de 1 à 20 % en poids de la composition.

6. Procédé selon l'une quelconque des revendications précédentes, caractérisée en ce que l'acide ascorbique est mis en oeuvre en une quantité allant de 3 à 10 % en poids de la composition.

7. Procédé selon l'une quelconque des revendications précédentes, caractérisée en ce que la composition est une solution aqueuse, huileuse ou hydroalcoolique, une émulsion eau-dans-huile, une émulsion huile-dans-eau, une micro-émulsion, un gel aqueux, un gel huileux, un gel anhydre, un sérum, une dispersion de vésicules.

8. Procédé selon l'une quelconque des revendications précédentes, caractérisée en ce que la composition comprend au moins un adjuvant lipophile ou hydrophile choisi parmi les conservateurs, les antioxydants, les agents chélateurs, les parfums, les charges, les filtres, les séquestrants, les huiles essentielles, les matières colorantes, les actifs hydrophiles ou lipophiles, et les vésicules lipidiques.

9. Procédé selon l'une quelconque des revendications précédentes, caractérisé en ce que la composition se présente sous forme d'un support et d'un solide distincts, destinés à être mélangés extemporanément, le solide consistant essentiellement en de l'acide ascorbique pulvérulent.

10. Utilisation de l'acide ascorbique sous forme libre comme seul actif pour la fabrication d'une composition dermatologique destinée à traiter la séborrhée de la peau et/ou du cuir chevelu.

11. Utilisation selon la revendication 10, caractérisée en ce que la composition a un pH allant de 2 à 5.

12. Utilisation selon la revendication 11, caractérisée en ce que la composition a un pH de 4.

13. Utilisation selon l'une quelconque des revendications 10-12, caractérisée en ce que l'acide ascorbique est mis en oeuvre en une quantité allant de 1 à 20 % en poids de la composition.

14. Utilisation selon l'une quelconque des revendications 10-13, caractérisée en ce que l'acide ascorbique est mis en oeuvre en une quantité allant de 3 à 10 % en poids de la composition.

15. Utilisation selon l'une quelconque des revendications 10-14, caractérisée en ce que la composition est une solution aqueuse, huileuse ou hydroalcoolique, une émulsion eau-dans-huile, une émulsion huile-dans-eau, une micro-émulsion, un gel aqueux, un gel huileux, un gel anhydre, un sérum, une dispersion de vésicules.

16. Utilisation selon l'une quelconque des revendications 10-15, caractérisée en ce que la composition comprend au moins un adjuvant lipophile ou hydrophile choisi parmi les conservateurs, les antioxydants, les agents chélateurs, les parfums, les charges, les filtres, les séquestrants, les huiles essentielles, les matières colorantes, les actifs hydrophiles ou lipophiles, et les vésicules lipidiques.

17. Utilisation selon l'une quelconque des revendications 10-16, caractérisé en ce que la composition se présente sous forme d'un support et d'un solide distincts, destinés à être mélangés extemporanément, le solide consistant essentiellement en de l'acide ascorbique pulvérulent.

## Claims

1. Process for the cosmetic treatment of seborrhoea, which consists in applying, to the skin and/or the scalp, a composition containing ascorbic acid as sole agent for treating seborrhoea.

2. Process according to Claim 1, characterized in that the ascorbic acid is in free form.

3. Process according to either one of Claims 1 and 2, characterized in that the composition has a pH ranging from 2 to 5.

4. Process according to any one of the preceding claims, characterized in that the composition has a pH of 4.

5. Process according to any one of the preceding claims, characterized in that the ascorbic acid is used in an amount ranging from 1 to 20 % by weight of the composition.

6. Process according to any one of the preceding claims, characterized in that the ascorbic acid is used in an amount ranging from 3 to 10 % by weight of the composition.

7. Process according to any one of the preceding claims, characterized in that the composition is an aqueous, oily or aqueous-alcoholic solution, a water-in-oil emulsion, an oil-in-water emulsion, a microemulsion, an aqueous gel, an oily gel, an anhydrous gel, a serum or a vesicle dispersion.

8. Process according to any one of the preceding claims, characterized in that the composition comprises at least one lipophilic or hydrophilic adjuvant chosen from preserving agents, antioxidants, chelating agents, fragrances, fillers, screening agents, sequestering agents, essential oils, dyestuffs, hydrophilic or lipophilic active agents, and lipid vesicles.

9. Process according to any one of the preceding claims, characterized in that the composition is in the form of a separate support and solid, which are intended to be mixed together at the time of use, the solid consisting essentially of pulverulent ascorbic acid.

10. Use of ascorbic acid in free form as sole active agent for manufacturing a dermatological composition intended for treating seborrhoea of the skin and/or the scalp.

11. Use according to Claim 10, characterized in that the composition has a pH ranging from 2 to 5.

12. Use according to Claim 11, characterized in that the composition has a pH of 4.

13. Use according to any one of Claims 10-12, characterized in that the ascorbic acid is used in an amount ranging from 1 to 20% by weight of the composition.

14. Use according to any one of Claims 10-13, characterized in that the ascorbic acid is used in an amount ranging from 3 to 10% by weight of the composition.

15. Use according to any one of Claims 10-14, characterized in that the composition is an aqueous, oily or aqueous-alcoholic solution, a water-in-oil emulsion, an oil-in-water emulsion, a microemulsion, an aqueous gel, an oily gel, an anhydrous gel, a serum or a vesicle dispersion.

16. Use according to any one of Claims 10-15, characterized in that the composition comprises at least one lipophilic or hydrophilic adjuvant chosen from preserving agents, antioxidants, chelating agents, fragrances, fillers, screening agents, sequestering agents, essential oils, dyestuffs, hydrophilic or lipophilic active agents, and lipid vesicles.

17. Use according to any one of Claims 10-16, characterized in that the composition is in the form of a separate support and solid, which are intended to be mixed together at the time of use, the solid consisting essentially of pulverulent ascorbic acid.

## Patentansprüche

1. Verfahren zur kosmetischen Behandlung der Seborrhoe, das darin besteht, auf die Haut und/oder die Kopfhaut eine Zusammensetzung aufzutragen, die Ascorbinsäure als einziges Mittel zur Behandlung der Seborrhoe enthält.

2. Verfahren nach Anspruch 1, dadurch gekennzeichnet, daß die Ascorbinsäure in freier Form vorliegt.

3. Verfahren nach einem der Ansprüche 1 oder 2, dadurch gekennzeichnet, daß die Zusammensetzung einen pH-Wert im Bereich von 2 bis 5 aufweist.

4. Verfahren nach einem der vorhergehenden Ansprüche, dadurch gekennzeichnet, daß die Zusammensetzung einen pH-Wert von 4 aufweist.

5. Verfahren nach einem der vorhergehenden Ansprüche, dadurch gekennzeichnet, daß die Ascorbinsäure in einem Mengenanteil von 1 bis 20 % des Gewichts der Zusammensetzung verwendet wird.

6. Verfahren nach einem der vorhergehenden Ansprüche, dadurch gekennzeichnet, daß die Ascorbinsäure in einem Mengenanteil von 3 bis 10 % des Gewichts der Zusammensetzung verwendet wird.

7. Verfahren nach einem der vorhergehenden Ansprüche, dadurch gekennzeichnet, daß es sich bei der Zusammensetzung um eine wäßrige Lösung, eine ölige Lösung, eine wäßrig-alkoholische Lösung, eine Wasser-in-Öl-Emulsion, eine Öl-in-Wasser-Emulsion, eine Mikroemulsion, ein wäßriges Gel, ein öliges Gel, ein wasserfreies Gel, ein Serum oder eine Vesikeldispersion handelt.

8. Verfahren nach einem der vorhergehenden Ansprüche, dadurch gekennzeichnet, daß die Zusammensetzung mindestens einen lipophilen oder hydrophilen Zusatzstoff enthält, der unter den Konservierungsmitteln, Antioxidantien, Chelatbildnern, Parfums, Füllstoffen, Filtern, Maskierungsmitteln, etherischen Ölen, Färbemitteln, hydrophilen oder lipophilen Wirkstoffen und Lipidvesikeln ausgewählt ist.

9. Verfahren nach einem der vorhergehenden Ansprüche, dadurch gekennzeichnet, daß die Zusammensetzung in Form eines Trägers und eines Feststoffs vorliegt, die voneinander verschieden sind und die kurz vor der Anwendung vermischt werden sollen, wobei der Feststoff im wesentlichen aus pulverförmiger Ascorbinsäure besteht.

10. Verwendung von Ascorbinsäure in freier Form als alleinigen Wirkstoff zur Herstellung einer dermatologischen Zusammensetzung, die zur Behandlung von Seborrhoe der Haut und/oder der Kopfhaut bestimmt ist.

11. Verwendung nach Anspruch 10, dadurch gekennzeichnet, daß die Zusammensetzung einen pH-Wert im Bereich von 2 bis 5 aufweist.

12. Verwendung nach Anspruch 11, dadurch gekennzeichnet, daß die Zusammensetzung einen pH-Wert von 4 aufweist.

13. Verwendung nach einem der Ansprüche 10 bis 12, dadurch gekennzeichnet, daß die Ascorbinsäure in einem Mengenanteil von 1 bis 20 % des Gewichts der Zusammensetzung verwendet wird.

14. Verwendung nach einem der Ansprüche 10 bis 13, dadurch gekennzeichnet, daß die Ascorbinsäure in einem Mengenanteil von 3 bis 10 % des Gewichts der Zusammensetzung verwendet wird.

15. Verwendung nach einem der Ansprüche 10 bis 14, dadurch gekennzeichnet, daß es sich bei der Zusammensetzung um eine wäßrige Lösung, eine ölige Lösung, eine wäßrig-alkoholische Lösung, eine Wasser-in-Öl-Emulsion, eine Öl-in-Wasser-Emulsion, eine Mikroemulsion, ein wäßriges Gel, ein öliges Gel, ein wasserfreies Gel, ein Serum oder eine Vesikeldispersion handelt.

16. Verwendung nach einem der Ansprüche 10 bis 15, dadurch gekennzeichnet, daß die Zusammensetzung mindestens einen lipophilen oder hydrophilen Zusatzstoff enthält, der unter den Konservierungsmitteln, Antioxidantien, Chelatbildnern, Parfums, Füllstoffen, Filtern, Maskierungsmitteln, etherischen Ölen, Färbemitteln, hydrophilen oder lipophilen Wirkstoffen und Lipidvesikeln ausgewählt ist.

17. Verwendung nach einem der Ansprüche 10 bis 16, dadurch gekennzeichnet, daß die Zusammensetzung in Form eines Trägers und eines Feststoffs vorliegt, die voneinander verschieden sind und die kurz vor der Anwendung vermischt werden sollen, wobei der Feststoff im wesentlichen aus pulverförmiger Ascorbinsäure besteht.
